# NEUE EUROPÄISCHE PATENTSCHRIFT

(11) **EP 2 411 762 B2**
(45) Veröffentlichungstag und Bekanntmachung des Hinweises auf die Entscheidung über den Einspruch: **23.10.2019**
(45) Hinweis auf die Patenterteilung: 30.11.2016
(21) Anmeldenummer: 10703183.3
(22) Anmeldetag: 05.02.2010
(51) Int. Cl.: G01C 15/00

(54) **SELBSTNIVELLIERENDES MEHR-LINIEN- 360°-LASERGERÄT**
SELF LEVELLING MULTI LINE 360° LASER DEVICE
APPAREIL LASER À 360°, À PLUSIEURS LIGNES ET À NIVELLEMENT AUTOMATIQUE

(30) Priorität: 26.03.2009 DE 102009001875
(43) Veröffentlichungstag der Anmeldung: 01.02.2012
(73) Patentinhaber: Robert Bosch GmbH, 70442 Stuttgart (DE)
(72) Erfinder: ZIMMERMANN, Thomas, 80639 Muenchen (DE)
(86) Internationale Anmeldenummer: PCT/EP2010/051403
(87) Internationale Veröffentlichungsnummer: WO 2010/108721

(56) Entgegenhaltungen:
- CA-C- 2 116 445
- CN-A- 1 884 970
- DE-A1-102004 045 808
- DE-T2- 60 202 114
- JP-A- 2002 195 831
- TW-U- 426 154
- TW-U- M 297 465
- US-A- 4 111 564
- US-A1- 2003 000 094
- US-A1- 2004 085 646
- US-A1- 2004 107 588
- US-A1- 2005 091 859

## Beschreibung

### Stand der Technik

Die Erfindung bezieht sich auf ein selbstnivellierendes Mehr-Linien-Lasergerät zur Erzeugung zumindest zweier, aufeinander senkrecht stehender Laserlinien, die jeweils über einen Winkelbereich von 360° projizierbar sind. Durch ein solches Gerät können beispielsweise horizontale und vertikale Ebenen durch jeweils eindimensional aufgeweitete Laserstrahlen aufgespannt werden und horizontale bzw. vertikale Linien über einen Winkelbereich von 360° beispielsweise auf Wandflächen oder Gegenstände projiziert werden. Diese projizierbaren Linien werden hinfort als Laserlinien oder projizierte Laserlinien des Mehr-Linien-Lasergerätes bezeichnet, obwohl das Gerät diese Linien nicht selbst, sondern nur durch Projektion des aufgeweiteten Laserstrahls beispielsweise auf eine Wand oder einen Gegenstand hervorbringen kann. Derartige Mehr-Linien-Lasergeräte können insbesondere in Industrie, Handwerk und Heimwerkerbereich beispielsweise für Justier-, Markier-, Mess- und Ausrichtaufgaben Verwendung finden.

Es sind aus dem Stand der Technik Rotationslasergeräte bekannt, bei denen ein Laserstrahl durch ein schnell rotierendes Umlenkelement im 90°-Winkel umgelenkt und so durch den mit dem rotierenden Umlenkelement umlaufenden Laserstrahl die optische Illusion einer kontinuierlichen Laserlinie erzeugt wird. Durch Kombination zweier im 90°-Winkel angeordneter Rotationslasereinheiten in einem Gerät lassen sich so zwei aufeinander senkrecht stehende Laserlinien beispielsweise auf Wände oder Gegenstände projizieren.

Nachteile solcher Rotationslasergeräte liegen u.a. in der aufwendigen mechanischen Konstruktion und den damit verbundenen hohen Herstellungskosten, sowie der großen, schweren Bauform derartiger Lasergeräte. Weitere Nachteile derartiger Geräte liegen in hohem Energieverbrauch und im Verschleiß sowie in der begrenzten Zuverlässigkeit über die Lebensdauer der Geräte.

Aus der US 2005/0091859A1 ist ein Laserstrahl-Modul zur gleichzeitigen Erzeugung zweier senkrecht zueinander stehender Lasereben bekannt. Das System der US 2005/0091859A1 erzeugt, ausgehend von einer Laserstrahlquelle durch Strahlteilung und Strahlumlenkung an prismatischen Glaskörpern senkrecht zueinander stehende Laserstrahlen. Durch Rotation dieser Anordnung mittels eines Hohlachsenmotors durch den das Lichtsignal hindurchgeführt wird, werden aus den Laserstrahlen aufgrund der Trägheit des menschlichen Auges Laserebenen ausgebildet. In einer speziellen Ausführungsform schlägt die US 2005/0091859A1 vor, die Totalreflexion an einem transparenten Negativkegel zu verwenden, um eine Laserebene ohne Rotation zu erzeugen.

Aus der US 4,111,564 A ist ein Konstruktionswerkzeug für die Erzeugung einer gleichmäßigen Lichtebene zur Verwendung als Bezugslinie bekannt. Die US 4,111,564 schlägt dazu vor, einen Bezugsebenenreflektor mit einen reflektierenden 45°-Konus zu verwenden, dessen Achse mit der Achse eines Lichtstrahlenbündels fluchtet. Wenn ein Lichtbündel auf die obere Oberfläche des Konus trifft, wird das Lichtbündel gleichzeitig in verschiedene Richtungen in eine dünne sich radial erstreckende Bezugsebene reflektiert. Die Dicke der derart erzeugten Bezugsebene liegt wünschenswerterweise im Bereich zwischen 1,6 mm bis 6,3 mm, obwohl nach der Lehre der US 4,111,564 A auch Dicken bis zu 25 mm verwendet werden können. Die Ebene des reflektierten Lichtes tritt mit wesentlicher Gleichförmigkeit unter 90° zum einfallenden Strahlenbündel aus. Das Lichtbündel kann von jeder gewünschten Frequenz im elektromagnetischen Spektrum sein, jedoch verwendet man im allgemeinsten Falle Frequenzen aus dem sichtbaren Lichtbereich. Bündeldurchmesser unter 50 mm und vorzugsweise unter 25 mm sind in bestimmten Fällen bevorzugt. Obwohl die besondere Form der Laserlichtbündelquelle unwesentlich ist, eignet sich nach der Lehre der US 4,111,564 A besonders ein Helium-Neon-Gas-Laser für die Zwecke der vorliegenden Erfindung. Die Strahlung im sichtbaren Rotteil des Spektrums ist sicher sowie leicht zu handhaben und stellt eine einfache Form einer Laserbündelprojektion dar.

### Aufgabe der Erfindung

Aufgabe der vorliegenden Erfindung ist es, die Nachteile des Standes der Technik zu überwinden und insbesondere ein selbstnivellierendes Mehr-Linien-Lasergerät bereitzustellen, das bei kleiner Bauform, günstigen Herstellungskosten und ohne rotierende Teile Laserlinien über einen Winkelbereich von 360° projizieren kann.

### Offenbarung der Erfindung

Die Aufgabe der vorliegenden Erfindung wird durch ein selbstnivellierendes Mehr-Linien-Lasergerät mit drei Linienprojektionsvorrichtungen gemäß Anspruch 1 gelöst, bei dem jede dieser Linienprojektionsvorrichtungen einen reflektierenden Kegel und einen Laserstrahl, der in Richtung der Kegelachse dieses reflektierenden Kegels gegen die Spitze dieses Kegels richtbar ist, aufweist, wobei die Linienprojektionsvorrichtungen zueinander senkrecht angeordnet sind, und wobei die Linienprojektionsvorrichtungen ferner zumindest abschnittsweise zylinderförmige, transparente Kegelträger aufweisen, welche zumindest abschnittsweise um die reflektierenden Kegel herum angeordnet sind, wobei jeder Kegelträger zumindest abschnittsweise eine rotationssymmetrische hohlzylindrische Form aufweist und zumindest abschnittsweise koaxial um den reflektierenden Kegel angeordnet ist, sowie die Laserquellen und die Linienprojektionsvorrichtungen an und/oder in einem Optikträger montiert sind, wobei der Optikträger pendelnd ausgeführt und an Lagerachsen aufgehängt ist, und die Achsen der reflektierenden Kegel aufeinander senkrecht stehen und Achsen eines dreidimensionalen Koordinatensystems aufspannen, wobei sich die Achsen der reflektierenden Kegel in einem Punkt schneiden, wobei sich die Laserstrahlen der drei Laserquellen alle in einem Punkt, der auf der Schwerpunktlinie durch den Aufhängungspunkt des Optikträgers liegt, kreuzen. Hierdurch ist es möglich, drei aufeinander senkrecht stehende Laserlinien zu erzeugen, welche jeweils über einen Winkelbereich von 360° projizierbar sind. Wird im Folgenden von der Ausrichtung einer Linienprojektionsvorrichtung gesprochen, so ist diese gegeben durch die Mittelachse des Laserstrahls, der gegen die Spitze des reflektierenden Kegels gerichtet ist, bzw. die Achse dieses Kegels selbst, wobei - im Rahmen des produktionstechnisch bzw. wirtschaftlich Möglichen - diese beiden Achsen parallel und idealerweise kollinear sind.

Des weiteren wird die Aufgabe erfindungsgemäß gelöst durch eine Linienprojektionsvorrichtung wobei die Linienprojektionsvorrichtung einen reflektierenden Kegel, um den herum zumindest abschnittsweise ein zumindest abschnittsweise zylinderförmiger, transparenter Kegelträger angeordnet ist, sowie einen Laserstrahl, der in Richtung der Achse dieses reflektierenden Kegels gegen die Spitze dieses Kegels richtbar ist, aufweist, wodurch dieser Laserstrahl zu einer projizierbaren 360°-Laserlinie aufweitbar ist.

Bevorzugte Ausführungsformen sind in den abhängigen Ansprüchen angegeben. Ein Vorteil der Erfindung liegt darin, dass sich durch das erfindungsgemäße Mehr-Linien-Lasergerät eine hohe Präzision und Langzeitstabilität bzgl. der Ausrichtung und Lagegenauigkeit der Laserlinien - auch im langjährigen Einsatz unter widrigen Einsatzbedingungen - erzielen lässt. Ein weiterer Vorzug besteht darin, dass das erfindungsgemäße Mehr-Linien-Lasergerät ohne bewegliche Teile in der optischen Konstruktion auskommt. Zudem lässt sich das erfindungsgemäße Mehr-Linien-Lasergerät ohne konstruktiven Aufwand und ohne große Mehrkosten um eine dritte Laserlinie erweitern, wodurch sich ein vollständiges Raum-Koordinatensystem durch das erfindungsgemäße Mehr-Linien-Lasergerät aufspannen lässt. Weiterhin vorteilhaft ist, dass durch das erfindungsgemäße Mehr-Linien-Lasergerät in den meisten Fällen auf den Einsatz der aufwendigen, teuren, schweren Rotationslasergeräte verzichtet werden kann. In einer bevorzugten Ausführungsform der Erfindung können die Linienprojektionsvorrichtungen ferner zumindest abschnittsweise zylinderförmige, transparente Kegelträger aufweisen, welche zumindest abschnittsweise um die reflektierenden Kegel herum angeordnet sind. Durch solche Kegelträger ist es möglich, die reflektierenden Kegel so an dem weiter unten beschriebenen Optikträger zu befestigen, dass der Laserstrahl durch den Kegelträger hindurch in Richtung der Kegelachse gegen die Spitze des reflektierenden Kegels gerichtet werden kann, dass der im 90°-Winkel umgelenkte Laserstrahl eine 360° Laserlinie erzeugen kann, wobei der umgelenkte Laserstrahl möglichst ungehindert durch den Kegelträger hindurchtreten kann. Dieser Kegelträger kann vorteilhafterweise aus einem transparenten Kunststoff (beispielsweise aus PMMA [Polymethylmethacrylat], PC [Polycarbonat], COC [Cyclo-Olefin-(Co)polymer]), aber auch aus "mineralischem" Glas hergestellt sein.

Entsprechend der Erfindung soll der Kegelträger zumindest abschnittsweise eine vorzugsweise rotationssymmetrische hohlzylindrische Form aufweisen und vorzugsweise zumindest abschnittsweise koaxial um den reflektierenden Kegel angeordnet sein. Hierdurch ist möglich, den reflektierenden Kegel mechanisch stabil zu halten, ohne den Strahlengang des auf den reflektierenden Kegel auftreffenden Laserstrahls, noch den der reflektierten Laserlinie optisch nachteilig zu beeinflussen.

In einer weiteren bevorzugten Ausführungsform der Erfindung kann der reflektierende Kegel zumindest abschnittsweise als gerader Kreiskegel mit 90° Kegelöffnungswinkel ausgebildet sein. Hierdurch kann ein parallelgerichteter Laserstrahl, der parallel zur Kegelachse des geraden Kreiskegels einfällt, exakt in einer Ebene aufgeweitet und so eine 360° Laserlinie erzeugt werden.

In einem weiteren Beispiel kann der reflektierende Kegel als Negativkegel - vorzugsweise einstückig mit dem Kegelträger - ausgebildet sein. Anstelle einer Kegel-Positivform ist es technisch genauso möglich, aus einem transparenten Materialblock beispielsweise aus Glas oder einem transparenten Polymer wie beispielsweise aus PMMA [Polymethylmethacrylat], PC [Polycarbonat], COC [Cyclo-Olefin-(Co)polymer] einen Kegel als "Negativform" auszusparen und vorzugsweise die Mantelfläche dieses Negativkegels zu verspiegeln. Bei dieser Ausführungsform wird dann der Laserstrahl durch den transparenten Materialblock hindurch gegen die Spitze des Negativkegels gerichtet. Hierdurch lässt sich dieselbe technische Wirkung der Laserstrahlaufweitung erzielen, wie bei einem Positivkegel. Besonders vorteilhaft kann ein solcher Kegel einstückig mit dem Kegelträger ausgebildet werden, der beispielsweise dann als kreiszylindrischer, transparenter Vollstab mit an einer Basisfläche ausgebildetem Negativkegel ausgeführt sein. Ein solches Bauteil kann beispielsweise in Großserie mit hoher Präzision in einem Urformverfahren, wie beispielsweise dem Spritzgießverfahren hergestellt werden. Hierdurch ist es möglich, die optische Funktion des Kegels und die mechanische Funktion des Kegelhalters in einem Bauteil zu vereinigen und hierdurch Produktions- und Montageaufwand und Kosten zu sparen. Zudem wäre es denkbar, einen solchen Glas- oder Kunststoffvollzylinder mit "eingeschliffenem" Negativkegel zusammen mit der Kollimatorlinse in einem Bauteil zu verwirklichen, wobei durch die runde Begrenzung der Kollimatorlinse zugleich die Maskierung des Lichtkegels der Laserdiode erfolgen - und somit die Verwendung einer separaten Kreisblende entfallen könnte.

In einer weiteren bevorzugten Ausführungsform der Erfindung kann zumindest ein Laserstrahl durch eine Laserdiode als Laserquelle erzeugbar sein, welcher vorzugsweise durch zumindest ein kollimierendes optisches Element, insbesondere eine Kollimatorlinse, kollimierbar ist. Durch den Einsatz von Laserdioden als Laserquellen ist eine besonders kostengünstige Herstellung sowie eine kompaktere Bauform des Mehr-Linien-Lasergerätes möglich. Mittels des kollimierenden optischen Elements kann dabei der divergente Laserstrahl, wie er von Laserdioden emittiert wird, kollimiert, also parallelgerichtet werden, wodurch eine exaktere Projektionsgeometrie und damit eine exaktere Laserlinie unter Verwendung eines geraden Kreiskegels erzielt werden kann.
In einer weiteren bevorzugten Ausführungsform der Erfindung kann eine Blende im Strahlengang der Laserdiode angeordnet sein. Durch eine solche Blende, die beispielsweise im optischen Strahlengang vor dem reflektierenden Kegel angeordnet sein kann, ist es möglich, beispielsweise den elliptischen Laserstrahl einer Laserdiode so zu formen, dass eine gleichmäßige Laserlinie über den gesamten Winkelbereich von 360° erzeugt wird und "Fehllicht", das nicht vom Kegelmantel umgelenkt wird, vermieden wird.

In einer weiteren bevorzugten Ausführungsform der Erfindung können zumindest zwei Laserstrahlen vorzugsweise mittels Strahlteilung durch ein teilreflektierendes optisches Element aus einer Laserquelle, vorzugsweise aus einer Laserdiode auskoppelbar sein. Dies kann im einfachsten Fall durch einen teildurchlässigen Spiegel erzielt werden, der im 45°-Winkel zu dem zu teilenden Laserstrahl angeordnet ist. Hierdurch ist es möglich, eine Laserquelle einzusparen. Dies ist insbesondere vorteilhaft, wenn beispielsweise eine grüne Laserlinie durch das Mehr-Linien-Lasergerät bereitgestellt werden soll, da derzeit im grünen Spektralbereich emittierende Laserquellen noch relativ hochpreisig sind.

In einer weiteren bevorzugten Ausführungsform der Erfindung kann ein Optikträger, an und/oder in dem die Linienprojektionsvorrichtungen montierbar sind, in einem Gravitationsfeld ausrichtbar - vorzugsweise selbstausrichtend - sein, wodurch die Laserstrahlen und Kegelachsen der Linienprojektionsvorrichtungen in Richtung des Gravitationsvektors oder senkrecht zu diesem ausrichtbar sind. Durch eine solche Konstruktion können besonders zweckmäßig und exakt horizontale und vertikale Laserlinien bereitgestellt werden.

In der Ausführungsform der Erfindung sind die Laserquelle(n) und die Linienprojektionsvorrichtungen an und/oder in einem Optikträger montierbar wobei der Optikträger selbstnivellierend ausgeführt und pendelnd an zwei zueinander senkrechten und in einem Betriebszustand im Wesentlichen horizontal ausgerichteten Lagerachsen aufgehängt. Hierdurch kann auf besonders vorteilhafte Weise eine Selbstnivellierfähigkeit des Mehr-Linien-Lasergerätes erreicht werden. In einer weiteren bevorzugten Ausführungsform der Erfindung kann der Optikträger eine Schwingungsdämpfung, vorzugsweise eine magnetische Dämpfung, insbesondere eine Wirbelstromdämpfung aufweisen. Durch eine solche Schwingungsdämpfung kann die Einschwingdauer und die erzielbare Einstellgenauigkeit der Laserlinien erheblich verbessert dadurch Praxisnutzen und Effizienz beim praktischen Routineeinsatz erhöht werden.

### Zeichnung

Anhand der Zeichnungen wird die Erfindung nachstehend exemplarisch anhand von Ausführungsbeispielen eingehend erläutert. Die Beschreibung, die zugehörigen Figuren sowie die Ansprüche enthalten zahlreiche Merkmale in Kombination. Ein Fachmann wird diese Merkmale, insbesondere auch die Merkmale verschiedener Ausführungsbeispiele, auch einzeln betrachten und zu sinnvollen, weiteren Kombinationen zusammenfassen.

Es zeigen:
- Fig. 1: eine perspektivische Aufsicht auf einen in der Achse zweier Linienprojektionsvorrichtungen aufgeschnittenen Optikträgers einer Ausführungsform eines selbstnivellierenden Drei-Linien-Lasergerätes mit eingebauten Lasermodulen (beinhaltend jeweils u.a. Laserdiode und Kollimatorlinse) und Projektionskegeln (reflektierenden Kegeln) mit Kegelträgern;
- Fig. 2: eine gerade Aufsicht auf den aufgeschnittenen Optikträger einer Ausführungsform als Zwei-Linien-Lasergerät mit den Ebenen 10a und 10c der Laserlinien und
- Fig. 3: eine Ansicht eines Drei-Linien-Lasergeräts mit dargestellten Ebenen der Laserlinien.

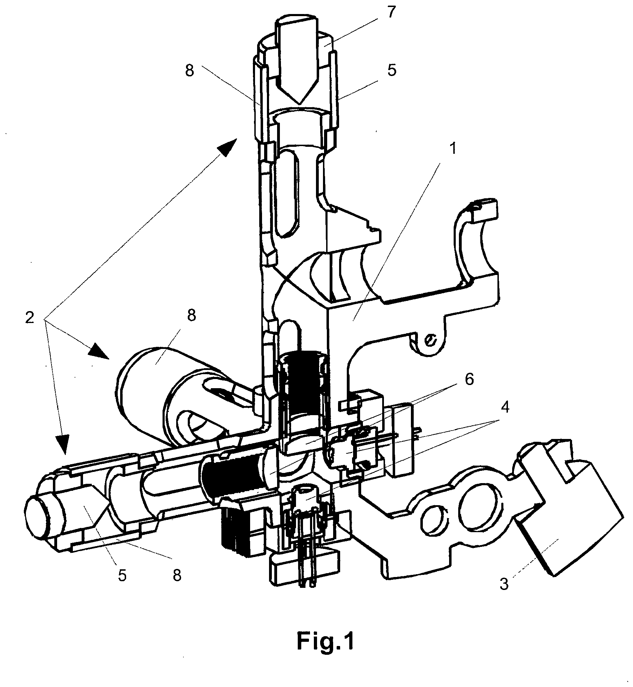

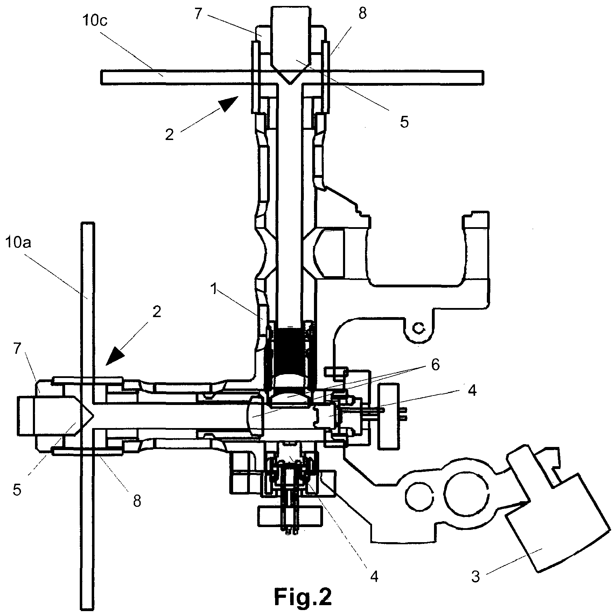

Die Darstellungen der Fig. 1 und 2 zeigen den grundlegenden Aufbau einer Ausführungsform des selbstnivellierenden Mehr-Linien-Lasergeräts mit dem Optikträger 1, bestückt mit den wesentlichen optischen Komponenten, wobei die hier dargestellte Ausführungsform des selbstnivellierenden Mehr-Linien-Lasergeräts mit drei Linienprojektionsvorrichtungen 2 ausgestattet ist. Hierbei zeigt Fig. 1 ein Drei-Linien-Lasergerät und Fig.2 ein Zwei-Linien-Lasergerät, wobei die Längsachsen der Laser jeweils senkrecht zueinander angeordnet sind. Der prinzipielle Unterschied von Fig. 1 zu Fig.2 besteht darin, dass in der Richtung, die in Fig.2 senkrecht zur Zeichenebene ist, kein Laser vorgesehen ist. Aus diesem Grund werden beide Ausführungsformen im Folgenden gemeinsam betrachtet.

Der Optikträger 1 besteht vorzugsweise im Wesentlichen aus einer metallischen Druckgusslegierung (vorzugsweise Aluminium- oder Zinkdruckgusslegierung) und trägt die wesentlichen optischen Elemente, die nachstehend erläutert werden. Dieser Optikträger 1 ist pendelnd in einem Rahmenelement (nicht dargestellt) aufgehängt, das mit einem Gerätegehäuse (nicht dargestellt) verbunden ist.

Die pendelnde Aufhängung des Optikträgers 1 im Rahmenelement erfolgt über ein Kreuzgelenk mit Kugellagern mittels einer zweiachsigen kardanischen Aufhängung, wobei die Lagerungsachsen für die Aufhängung des Optikträgers 1 jeweils beiderseits des Aufhängungspunktes in Kugellagern geführt sind. Zur Vermeidung von durch Alignierungstoleranzen bedingten Verspannungen in den Lagerungsachsen sind diese im Bereich der Kugellager-Innenhülse leicht ballig ausgeführt, um Winkeltoleranzen beispielsweise bei der Fertigung aufzufangen. Durch diesen Aufbau kann sich der Optikträger 1 um zwei Achsen frei auspendeln und entlang seiner Schwerpunktlinie im Gravitationsfeld der Erde ausrichten.

Um die Einschwingdauer des pendelnd und - im Rahmen des technisch Möglichen - weitestgehend reibungsfrei aufgehängten Optikträgers 1 erheblich zu verkürzen, ohne die Einstellgenauigkeit der Endposition des Optikträgers 1 im Gravitationsfeld zu verringern, besitzt der Optikträger 1 des hier dargestellten Ausführungsbeispiels eine Wirbelstromdämpfung nach dem Prinzip des Waltenhof'schen Pendels. Hierzu ist ein Kupferblock 5 am unteren, freien Ende des pendelnd aufgehängten Optikträgers 1 angebracht, wobei sich der Kupferblock 5 im Falle einer Pendelbewegung in geringem Abstand berührungsfrei über einen Permanentmagneten (nicht dargestellt), der mit dem Gehäuse 3 fest verbunden ist, bewegt. Der Kupferblock 5 ist zudem - wie aus den Figuren zu ersehen - aus der Schwerpunktachse des bestückten Optikträgers 1 herausgerückt und in einer Winkelstellung und mit einer Achsneigung des zylindrischen Kupferblocks 5 von ca. 20° bis 30° an einem Ausleger am im Betriebszustand unteren Ende des Optikträgers 1 angebracht. Hierdurch kann die Masse des Kupferblocks 3 - neben der Funktion als Bauteil der Wirbelstromdämpfung zur Dämpfung der Pendelbewegung des Optikträgers 1 - gleichzeitig als Ausgleichsgewicht zur Kompensation der weit aus der Schwerpunktlinie des Optikträgers 1 herausragenden Linienprojektionsvorrichtungen 2 zur Erzeugung der beiden in vertikalen Ebenen aufgeweiteten Laserstrahlen dienen.

Der vorerwähnte Permanentmagnet (nicht dargestellt) umfasst vorteilhafterweise eine Mehrzahl einzelner Magnetelemente (in diesem Fall: vier) mit vorzugsweise alternierender Magnetfeldorientierung und ist dahingehend optimiert, dass die Magnetfeldlinien mit einer möglichst hohen Magnetfelddichte und möglichst großen Magnetfeldstärkegradienten durch den Kupferblock 3 hindurchtreten und dort via elektromagnetischer Induktion ein dem Feld des Permanentmagneten entgegen gerichtetes Magnetfeld erzeugen; dieses induzierte Magnetfeld dämpft die Pendelbewegung des Optikträgers 1 stark, ohne jedoch die Endposition des Optikträgers 1 zu beeinflussen, da die Dämpfung nach dem magnetischen Wirbelstromprinzip proportional zur Relativbewegungsgeschwindigkeit zwischen Permanentmagnet und Kupferblock 3 ist und somit im statischen Falle nach Dämpfung der Bewegung des Optikträgers 1 entfällt.

Die Dämpfung kann auf diese Weise optimiert und beispielsweise nahezu auf den "aperiodischen Grenzfall" eingestellt werden. Mittels der Wirbelstromdämpfung stellt sich der Optikträger 1 nach dem Aufstellen des Drei-Linien-Lasergerätes oder nach einem Stoß gegen das Drei-Linien-Lasergerät innerhalb kurzer Zeit von typischerweise von 0,5 bis 5 Sekunden mit einer hohen Genauigkeit von z.B. wenigen Zehntel Millimetern pro Meter im Gravitationsfeld der Erde ein. Um die genaue Ausrichtung des Optikträgers 1 im Erdmagnetfeld zu justieren, kann der Optikträger in der Nähe des Kupferblocks zwei Tarierschrauben aufweisen (z.B. Madenschrauben; nicht dargestellt), die zueinander und zur Schwerpunktlinie des Optikträgers 1 vorzugsweise 90°-Winkel - sowie vorzugsweise zur Ausrichtung eines horizontalen Laserstrahls 0°-bzw. 90°-Winkel - einschließen und über deren Einschraubtiefe die Schwerpunktlage des Optikträgers 1 und hierdurch die exakte Ausrichtung der Laserlinien geringfügig korrigiert werden kann.

Ferner besitzt der Permanentmagnet, der einen größeren Durchmesser aufweist als der Kupferblock 3, an dessen Außenumfang einen Radialanschlag (nicht dargestellt) für besagten Kupferblock 3, wodurch die Pendelbewegung des Optikträgers 1 (und damit der Selbstnivellierbereich des selbstnivellierenden Drei-Linien-Lasergerätes) beispielsweise auf einen bestimmten Wert (beispielsweise im Bereich von 5° bis 15°, typischerweise auf maximal 5° oder 8°) begrenzt wird. Hierdurch kann ein Anschlagen des Optikträgers 1 oder der damit verbundenen Bauteile am Gehäuse und ein Überdehnen beispielsweise der (hochflexiblen und biegeschlaffen, jedoch äußerst dünnen und zugempfindlichen) elektrischen Versorgungskabel (nicht dargestellt) für die Laserquellen 4 beispielsweise bei sehr schräger Aufstellung des Drei-Linien-Lasergerätes, im Betrieb oder beim Transport verhindert werden. Bei Berührung zwischen dem Kupferblock 3 und dem Radialanschlag des Permanentmagneten 6 kann ein elektrischer Kontakt in einem Kontrollstromkreis geschlossen und der Benutzer so vor einem nicht korrekt nivellierten System z.B. durch ein optisches oder akustisches Signal oder durch periodisches Dunkeltasten (Blinken) der Laserquellen 4 gewarnt werden.

Der optische Aufbau weist in diesem Ausführungsbeispiel drei Linienprojektionsvorrichtungen 2 mit drei Laserstrahlquellen 4 auf, die im 90°-Winkel zueinander in den Richtungen der drei Raumachsen im Optikträger 1 verbaut sind, wobei sich die Laserstrahlen der drei Laserquellen 4 alle in einem Punkt, der hier auf der Schwerpunktlinie durch den Aufhängungspunkt des Optikträgers 1 liegt, kreuzen. Jeder der Laserstrahlen einer Linienprojektionsvorrichtung 2 ist gegen die Spitze eines Spiegelkegels (oder reflektierenden Kegels) 5 gerichtet, wodurch der Laserstrahl in einer zur Strahlrichtung senkrechten Teilebene 10a, 10b, 10c abgelenkt und zu einer 360°-Linie aufgeweitet wird, wobei die Teilebenen 10a, 10b, 10c der drei aufgeweiteten Laserstrahlen zueinander exakt 90°-Winkel einschließen. Dabei stehen auch die Achsen der reflektierenden Kegel 5, die kollinear zu den Laserstrahlen ausgerichtet sind, aufeinander senkrecht und spannen so Achsen eines dreidimensionalen Koordinatensystems auf; zudem schneiden sich in diesem Ausführungsbeispiel die Achsen der reflektierenden Kegel 5 - ebenso wie die der korrespondierenden Laserstrahlen - in einem Punkt, durch den auch die Schwerpunktlinie durch den Aufhängungspunkt des Optikträgers 1 hindurchgeht. In einer alternativen hier nicht dargestellten Ausführungsform können einzelne Laserquellen 4 entfallen, wobei beispielsweise ein Laserstrahl durch zwei teilreflektierende Strahlteiler in drei Laserteilstrahlen aufgespalten wird, die ihrerseits gegen die Spitzen der reflektierenden Kegel 5 gerichtet sind. Als Laserstrahlquellen kommen in diesem Ausführungsbeispiel preisgünstige low-power Laserdioden 4 zum Einsatz, die inhärent, d.h. aufgrund des physikalischen Entstehungsprinzips elliptisch divergente Lichtkegel erzeugen. Diese Lichtkegel werden zunächst durch Kollimatorlinsen 6 parallelgerichtet und danach gegebenenfalls durch Kreisblenden (nicht dargestellt) rund maskiert. Da der Justierung zwischen Laserdiode und Kollimatorlinse eine wesentliche Bedeutung zukommt, ist es unter produktionstechnischen und Kostengesichtspunkten vorteilhaft, Laserdiode 4 und Kollimatorlinse 6 zu einem Lasermodul als Baugruppe zusammenzufassen und ggf. auch die Blende in das Lasermodul zu integrieren, so dass die Lasermodule bereits ein parallelgerichtetes, rundes Strahlenbündel emittieren, und diese Lasermodule so - als vorgefertigte Baugruppen - in den Optikträger 1 einzubauen.
Jeder dieser runden Parallelstrahlen eines Lasermoduls 4 wird konzentrisch in Axialrichtung gegen die Spitze eines der Spiegelkegel 5, der die Form eines geraden Kreiskegels mit 90° Öffnungswinkel aufweist, gerichtet (d.h. die Mittelachse des Laserstrahls ist in Bezug auf die Kegelachse kollinear ausgerichtet), und beleuchtet den apikalen Abschnitt des reflektierenden Kegels 5 symmetrisch. Durch die verspiegelte, im 45°-Winkel gegen den Laserstrahl geneigte Mantelfläche des reflektierenden Kegels 5 wird der Laserstrahl im 90°-Winkel durch Reflexion umgelenkt und erzeugt so eine 360°-Vollkreisebene. Jeder dieser reflektierenden Kegel 5 muss einerseits so in Bezug auf den Optikträger 1 in Position gehalten werden, dass die Kegelspitze gegen den vom Lasermodul emittierten Laserstrahl gerichtet ist, wobei die Achse des reflektierenden Kegels 5 mit der Mittelachse des Laserstrahls vorzugsweise kollinear ist, also mit dieser auf einer Linie liegt; gleichzeitig darf aber die Abstrahlung des vom Kegelmantel reflektierten Laserlichts im 360°-Winkel nicht behindert werden. Zu diesem Zwecke sind die reflektieren Kegel 5, die in diesem Ausführungsbeispiel als zylindrische Stäbe mit zu geraden Kreiskegeln mit 90° Öffnungswinkel angespitzten und verspiegelten Enden ausgeführt sind, in Fassungsbuchsen 7 aufgenommen, welche ihrerseits in einem Kreiszylinderrohrabschnitt 8 aus einem transparenten Werkstoff, vorzugsweise aus einem Polymer wie PMMA [Polymethylmethacrylat], PC [Polycarbonat], COC [Cyclo-Olefin-(Co)polymer], montiert sind. Dabei ist die Länge des Rohrzylinders 8 größer als die Höhe des reflektierenden Kegelabschnitts 5 und überragt diese an beiden Rohrenden, so dass ein Teil dieses Überstandes zur Verbindung mit Optikträger 1 und Fassungsbuchse 7 zur exakten Radialpassung genutzt werden kann. Der reflektierende Kegelabschnitts 5 ist dabei vollständig und koaxial von dem transparenten Rohrzylinder 8 umgeben; das von dem Kegelmantel reflektierte und im 360°-Winkel in einer Ebene abgestrahlte Laserlicht kann somit ungehindert exakt in Radialrichtung durch die Wandung des transparenten Rohrzylinders 8 hindurch treten und - ohne optische Beeinträchtigungen, wie Verzerrungen oder Abschattungen -, eine im Winkelbereich von 360° projizierbare Laserlinie erzeugen. Gleichzeitig ermöglicht der transparente Rohrzylinder 8 ein hermetisches Verschließen des Hohlraums des Optikträgers 1 mit den optischen Komponenten der Linienprojektionsvorrichtung 2 mit den reflektierenden Kegeln 5, sowie den Lasermodulen mit Laserquelle 4, Kollimatorlinse 6 und Kreisblende. Auf diese Weise sind die empfindlichen optischen Oberflächen vor Feuchtigkeit, Staub und anderen widrigen Umwelteinflüssen im rauen Alltagseinsatz geschützt.

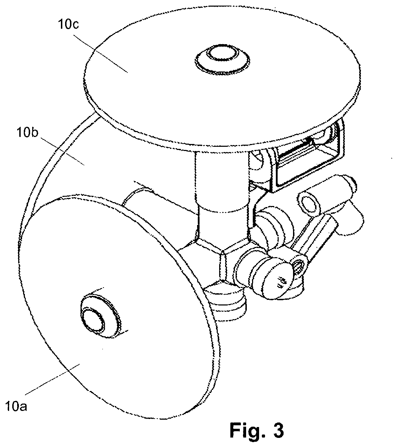

Durch das gemäß Fig. 1 und Fig.3 dargestellte Ausführungsbeispiel eines Drei-Linien-Lasergerätes mit dreimal 360°-Abdeckung der erzeugten Laserlinien können somit drei vollständige Raumebenen ausgezeichnet und ein räumliches Koordinatensystem mit Koordinatenachsen in sechs Raumrichtungen (±x-, ±y-und ±z-Richtung) aufgespannt werden. Die Ebenen der Laserlinien sind als 10a, 10b und 10c bezeichnet.

Grundsätzlich besteht ein Zusammenhang zwischen der Leistung der Laserdiode 4 einer bestimmten Bauart und der Elliptizität des emittierten Laserstrahls; so weisen insbesondere Laserstrahlen von Laserdioden 4 mit geringer Leistung besonders stark abgeflacht elliptische Strahlquerschnitte auf. Es kann daher vorteilhaft sein, insbesondere bei solchen Laserdioden 4 den Strahl geringfügig in den Randbereichen zu maskieren, so dass beispielsweise die äußersten Randbereiche in Richtung der langen Halbachse beschnitten werden und so ein Strahlquerschnitt entsteht, der weniger stark elliptisch oder sogar rund ist. Hierdurch kann beispielsweise die Helligkeitsverteilung der projizierbaren Laserlinie über den gesamten Winkelbereich von 360° noch besser ausgeglichen werden und zusätzlich verhindert werden, dass Teilbereiche des elliptischen Laserstrahls unreflektiert am reflektierenden Kegel 5 vorbei gehen und störende Reflexe oder Lichtfiguren erzeugen oder eine gefährliche Blendung von Personen hervorrufen. In einer vorteilhaften Ausführungsform kann der elliptische Laserstrahl jedoch auch ohne Maskierung verwendet werden. Hierbei wird die anisotrope Helligkeitsverteilung des Laserstrahls ausgenutzt und die lange Halbachse der Ellipse so ausgerichtet, dass die Helligkeitsmaxima des aufgeweiteten Laserstrahls in den gewünschten Richtungen orientiert sind. So ist es beispielsweise denkbar, den elliptischen Laserstrahl zur Erzeugung der horizontalen Laserlinie so auszurichten, dass die größte Helligkeit bzgl. des Gerätegehäuses nach "vorne" und nach "hinten" abgestrahlt wird. Ist das Linienlasergerät zum Einsatz in großen Hallen ausgelegt, so kann es zweckmäßig sein, die Helligkeitsmaxima der vertikalen Laserlinien "zu den Seiten zu" auszurichten, um eine ausreichende Helligkeit der Laserlinie auf weit entfernten Wänden zu erzeugen. In anderen Fällen kann eine andere Ausrichtung der Helligkeitsmaxima vorteilhaft sein. Es wäre auch denkbar, das Gerät so zu konstruieren, dass die lange Halbachse des elliptischen Laserstrahls je nach Einsatzgebiet und/oder Anwendungsfall gedreht werden kann, um so die Helligkeitsverteilung der Laserlinie Einsatzgebiet und/oder Anwendungsfall anzupassen.

Das Gerätegehäuse besteht vorzugsweise im Wesentlichen aus einem polymeren Werkstoff oder beispielsweise einem faserarmierten Polymer-Verbundwerkstoff (z.B. faserverstärkter Duroplast oder Thermoplast). Das Gerätegehäuse umgibt die zuvor beschriebene mechanische und optische Anordnung, schützt diese vor mechanischer Beschädigung und vermindert die Gefahr von Verunreinigungen. Das Gerätegehäuse weist drei Öffnungen auf, durch die die kegelbestückten Enden der Linienprojektionsvorrichtungen 2 hindurch treten und nach außen vorragen, so dass die durch die reflektierenden Kegel 5 aufgeweiteten Laserstrahlen ungehindert im 360°-Winkel abgestrahlt werden können. Die Gehäuseöffnungen sind so bemessen, dass die Beweglichkeit und Nivellierfähigkeit des Optikträgers 1 innerhalb des Selbstnivellierbereiches des selbstnivellierenden Drei-Linien-Lasergerätes nicht beeinträchtigt ist.

Weiterhin nimmt das Gehäuse in dem hier dargestellten Ausführungsbeispiel noch Batterien oder Ackus zur Stromversorgung (nicht dargestellt), Betätigungselemente, vorzugsweise Folientaster (nicht dargestellt) zum gemeinsamen, paarweisen und getrennten Schalten der drei Laserdioden 4, sowie eine elektronische Schaltung (nicht dargestellt) zum Betrieb der Laserdioden 4 auf. Die Stromversorgung der Laserquellen von der im Gehäuse montierten elektronischen Schaltung zu den im Optikträger 1 pendelnd aufgehängten Laserdioden 4 erfolgt über sehr dünne, hochflexible und biegeschlaffe elektrische Versorgungskabel (nicht dargestellt), die nahe am Aufhängungspunkt des Optikträgers 1 vorbei geführt sind, um das Auspendeln des Optikträgers 1 im Gravitationsfeld und die Nivelliergenauigkeit möglichst wenig zu beeinträchtigen.

Die Erfindung wird nicht durch konkrete Ausführungsformen begrenzt und Merkmale unterschiedlicher Ausführungsformen sind frei miteinander kombinierbar. Begriffe in der Anmeldung, die die Lage unterschiedlicher Komponenten zueinander beschreiben, wie "exakt 90°" oder "senkrecht zueinander", "auf einer Linie", "innerhalb der optischen Ebene" oder ähnliche beschreiben die gewünschte Idealposition/-lage und beinhaltet, dass sich aufgrund der mechanischen/optischen Ausgestaltung gewisse Abweichungen und Ungenauigkeiten ergeben können, die von der erfinderischen Lehre mit umfasst sind. Im Falle von Bereichsangaben sind nicht nur die angegebenen Endwerte, sondern auch alle dazwischen liegenden Werte und darin enthaltenen Teilbereiche von der erfinderischen Lehre mit umfasst. Wird in dieser Anmeldung von einer Laserlinie oder projizierbaren Laserlinie gesprochen, so ist hierbei die geometrische Figur gemeint, die entsteht, wenn der durch die Reflexion am Kegel in einer Ebene aufgeweitete Laserstrahl auf einen ebenen Gegenstand fällt und dort in der Projektion eine Laserlichtlinie erzeugt.

## Patentansprüche

1. Selbstnivellierendes Mehr-Linien-Lasergerät mit drei Linienprojektionsvorrichtungen (2), wobei jede dieser Linienprojektionsvorrichtungen (2) einen reflektierenden Kegel (5) und eine einen Laserstrahl erzeugende Laserquelle (4) aufweist und der in Richtung der Kegelachse dieses reflektierenden Kegels (5) gegen die Spitze dieses Kegels (5) richtbar ist, aufweist, wobei die Linienprojektionsvorrichtungen (2) zueinander senkrecht angeordnet sind und die Linienprojektionsvorrichtungen (2) ferner zumindest abschnittsweise zylinderförmige, transparente Kegelträger (8) aufweisen, welche zumindest abschnittsweise um die reflektierenden Kegel (5) herum angeordnet sind, wobei jeder Kegelträger (8) zumindest abschnittsweise eine rotationssymmetrische hohlzylindrische Form aufweist und zumindest abschnittsweise koaxial um den reflektierenden Kegel (5) angeordnet ist, **dadurch gekennzeichnet, dass** die Laserquellen (4) und die Linienprojektionsvorrichtungen (2) an und/oder in einem Optikträger (1) montiert sind, wobei der Optikträger (1) pendelnd ausgeführt und an Lagerachsen aufgehängt ist, und die Achsen der reflektierenden Kegel (5) aufeinander senkrecht stehen und Achsen eines dreidimensionalen Koordinatensystems aufspannen, wobei sich die Achsen der reflektierenden Kegel (5) in einem Punkt schneiden, wobei sich die Laserstrahlen der drei Laserquellen (4) alle in einem Punkt, der auf der Schwerpunktlinie durch den Aufhängungspunkt des Optikträgers (1) liegt, kreuzen.

2. Selbstnivellierendes Mehr-Linien-Lasergerät nach Anspruch 1, **dadurch gekennzeichnet, dass** der reflektierende Kegel (5) zumindest abschnittsweise als gerader Kreiskegel mit 90° Kegelöffnungswinkel ausgebildet ist.

3. Selbstnivellierendes Mehr-Linien-Lasergerät nach mindestens einem der vorhergehenden Ansprüche, **dadurch gekennzeichnet, dass** zumindest ein Laserstrahl durch eine Laserdiode (4) als Laserquelle erzeugbar ist, welcher vorzugsweise durch zumindest ein kollimierendes optisches Element, insbesondere eine Kollimatorlinse (6), kollimierbar ist.

4. Selbstnivellierendes Mehr-Linien-Lasergerät nach mindestens einem der vorhergehenden Ansprüche, **dadurch gekennzeichnet, dass** eine Blende im Strahlengang der Laserdiode (4) angeordnet ist.

5. Selbstnivellierendes Mehr-Linien-Lasergerät nach mindestens einem der vorhergehenden Ansprüche, **dadurch gekennzeichnet, dass** zumindest zwei Laserstrahlen vorzugsweise mittels Strahlteilung durch ein teilreflektierendes optisches Element aus einer Laserquelle, vorzugsweise aus einer Laserdiode (4) auskoppelbar sind.

6. Selbstnivellierendes Mehr-Linien-Lasergerät nach mindestens einem der vorhergehenden Ansprüche, **dadurch gekennzeichnet, dass** ein Optikträger (1), an und/oder in dem die Linienprojektionsvorrichtungen (2) montierbar sind, in einem Gravitationsfeld ausrichtbar - vorzugsweise selbstausrichtend - ist, wodurch die Laserstrahlen und Kegelachsen der Linienprojektionsvorrichtungen (2) in Richtung des Gravitationsvektors oder senkrecht zu diesem ausrichtbar sind.

7. Selbstnivellierendes Mehr-Linien-Lasergerät nach mindestens einem der vorhergehenden Ansprüche **dadurch gekennzeichnet, dass** der Optikträger (1) eine Schwingungsdämpfung, vorzugsweise eine magnetische Dämpfung, insbesondere eine Wirbelstromdämpfung (3) aufweist.

## Claims

1. Self-levelling multi-line laser device having three line projection devices (2), wherein each of said line projection devices (2) has a reflecting cone (5) and a laser source (4) generating a laser beam and which has is directable in the direction of the cone axis of this reflecting cone (5) at the tip of said cone (5), wherein the line projection devices (2) are arranged perpendicularly with respect to one another and the line projection devices (2) furthermore at least sectionally have cylindrical, transparent cone carriers (8) which are arranged at least sectionally around the reflecting cones (5), wherein each cone carrier (8) has at least sectionally a rotationally symmetrical hollow-cylindrical shape and is arranged at least sectionally coaxially about the reflecting cone (5), **characterized in that** the laser sources (4) and the line projection devices (2) are mounted on and/or in an optics carrier (1), wherein the optics carrier (1) is configured so that it oscillates and is suspended on bearing axes, and the axes of the reflecting cones (5) are perpendicular to each other and define axes of a three-dimensional coordinate system, wherein the axes of the reflecting cones (5) intersect in a point, wherein the laser beams of the three laser sources (4) all cross in a point that is located on the line of gravitation through the suspension point of the optics carrier (1).

2. Self-levelling multi-line laser device according to Claim 1, **characterized in that** the reflecting cone (5) is configured at least sectionally in the form of a straight circular cone with a 90° cone opening angle.

3. Self-levelling multi-line laser device according to at least one of the preceding claims, **characterized in that** at least one laser beam is generatable by a laser diode (4) as the laser source, which laser beam is collimatable preferably by at least one collimating optical element, in particular a collimator lens (6).

4. Self-levelling multi-line laser device according to at least one of the preceding claims, **characterized in that** a stop is arranged in the beam path of the laser diode (4).

5. Self-levelling multi-line laser device according to at least one of the preceding claims, **characterized in that** at least two laser beams are capable of being coupled out of a laser source, preferably out of a laser diode (4), preferably by means of beam splitting using a partially reflecting optical element.

6. Self-levelling multi-line laser device according to at least one of the preceding claims, **characterized in that** an optics carrier (1), on and/or in which the line projection devices (2) are mountable, is alignable in a gravitational field, preferably self-aligning, as a result of which the laser beams and cone axes of the line projection devices (2) are alignable in the direction of the gravitational vector or perpendicular thereto.

7. Self-levelling multi-line laser device according to at least one of the preceding claims, **characterized in that** the optics carrier (1) has vibration damping, preferably magnetic damping, in particular eddy current damping (3).

## Revendications

1. Appareil laser à lignes multiples à nivellement automatique comportant trois dispositifs de projection de lignes (2), dans lequel chacun desdits dispositifs de projection de lignes (2) comporte un cône réfléchissant (5) et une source laser (4) générant un faisceau laser et qui comporte peut être orienté dans la direction de l'axe de cône dudit cône réfléchissant (5) vers le sommet dudit cône (5), dans lequel les dispositifs de projection de lignes (2) sont disposés perpendiculairement les uns aux autres et les dispositifs de projection de lignes (2) comportent en outre des supports de cônes (8) transparents au moins partiellement cylindriques qui sont disposés au moins par sections autour du cône réfléchissant (5), dans lequel chaque support de cône (8) présente au moins par sections une forme cylindrique creuse symétrique de rotation et est disposé au moins par sections de manière coaxiale autour du cône réfléchissant (5), **caractérisé en ce que** les sources laser (4) et les dispositifs de projection de lignes (2) sont montés sur et/ou dans un support optique (1), le support optique (1) étant réalisé de manière à être oscillant et étant suspendu à des axes d'appui et les axes des cônes réfléchissants (5) étant perpendiculaires entre eux et définissant des axes d'un système de coordonnées tridimensionnel, les axes des cônes réfléchissants (5) passant par un point, les faisceaux laser des trois sources laser (4) s'intersectant tous en un point qui est situé sur la ligne du centre de gravité passant par le point de suspension du support optique (1).

2. Appareil laser à lignes multiples à nivellement automatique selon la revendication 1, **caractérisé en ce que** le cône réfléchissant (5) est réalisé au moins par sections sous la forme d'un cône circulaire droit ayant un angle d'ouverture du cône de 90°.

3. Appareil laser à lignes multiples à nivellement automatique selon au moins l'une des revendications précédentes, **caractérisé en ce qu'**au moins un faisceau laser peut être généré par une diode laser (4) en tant que source laser, lequel peut de préférence être collimaté par au moins un élément optique de collimation, notamment une lentille de collimation (6).

4. Appareil laser à lignes multiples à nivellement automatique selon au moins l'une des revendications précédentes, **caractérisé en ce qu'**un diaphragme est disposé sur le chemin de faisceau de la diode laser (4) .

5. Appareil laser à lignes multiples à nivellement automatique selon au moins l'une des revendications précédentes, **caractérisé en ce qu'**au moins deux faisceaux laser peuvent être couplés en sortie d'une source laser, de préférence d'une diode laser (4), de préférence au moyen d'un diviseur de faisceau, à travers un élément optique partiellement réfléchissant.

6. Appareil laser à lignes multiples à nivellement automatique selon au moins l'une des revendications précédentes, **caractérisé en ce qu'**un support optique (1) sur et/ou dans lequel peuvent être montés les dispositifs de projection de lignes (2), peut être orienté dans un champ gravitationnel - en s'orientant de préférence automatiquement -, les faisceaux laser et les axes de cône des dispositifs de projection de lignes (2) pouvant ainsi être orientés dans la direction du vecteur gravitationnel ou perpendiculairement à celui-ci.

7. Appareil laser à lignes multiples à nivellement automatique selon au moins l'une des revendications précédentes, **caractérisé en ce que** le support optique (1) comporte un dispositif d'amortissement des oscillations, de préférence un dispositif d'amortissement magnétique, notamment un dispositif d'amortissement à courants de Foucault (3).
